# EUROPEAN PATENT SPECIFICATION

(11) **EP 3 551 075 B1**
(45) Date of publication and mention of the grant of the patent: **07.06.2023**
(21) Application number: 17816611.2
(22) Date of filing: 05.12.2017
(51) Int. Cl.: A61B 5/087, A61B 5/113, A61B 5/00

(54) **SYSTEM AND METHOD FOR FACILITATING DETECTION OF A RESPIRATORY STATUS**
SYSTEM UND VERFAHREN ZUR ERMÖGLICHUNG DER DETEKTION EINES ATEMZUSTANDS
SYSTÈME ET PROCÉDÉ FACILITANT LA DÉTECTION D'UN ÉTAT RESPIRATOIRE

(30) Priority: 08.12.2016 EP 16020485
(43) Date of publication of application: 16.10.2019
(73) Proprietor: Boehringer Ingelheim International GmbH, 55216 Ingelheim am Rhein (DE)
(72) Inventor: ORSATTI, Leticia Moreira Goulart, 55216 Ingelheim am Rhein (DE); FERREIRA, Roberto Slepetys, 55216 Ingelheim am Rhein (DE)
(74) Representative: Weyers, Christopher
(86) International application number: PCT/EP2017/081535
(87) International publication number: WO 2018/104308

(56) References cited:
- WO-A1-2006/043278
- WO-A1-2014/128090
- WO-A1-2016/053897
- US-A- 4 909 260
- US-A- 4 909 260
- US-A- 6 019 732
- US-A1- 2002 072 685
- US-A1- 2007 299 325
- US-A1- 2007 299 325
- US-A1- 2011 301 485
- US-A1- 2012 041 279
- US-A1- 2012 190 989
- US-A1- 2013 317 333
- US-A1- 2014 121 473
- US-A1- 2014 121 473
- US-A1- 2015 087 922
- US-A1- 2015 126 822
- US-A1- 2015 126 822

## Description

The present invention relates to a system for facilitating detection of a respiratory status and a method for facilitating detection of a respiratory status.

The present invention generally relates to determination of properties of or depending on a respiratory tract, its tidal dynamics, its physiology and/or its geometry, the properties also being referred to as respiratory status.

The present invention in particular relates to the field of identifying or facilitating identification of the respiratory status in order to enable an assessment or a diagnosis with regard to respiratory diseases like asthma, but not limited to, where the respiratory tract is obstructed or reduced in airflow capacity.

Further, the present invention relates to wearable sensor devices. Such wearable sensor devices are known from long term examination of patients, for example with regard to long term blood pressure measurement or electrocardiography.

WO 2016/053897 A1 relates to a wearable device which is configured for facilitating diagnosis and/or assessment of pulmonary diseases. This device includes a flexible patch having an acoustic sensor, a motion sensor, a muscle activity sensor, a clock, an ambient temperature sensor, a communications port, a power source, a switching mechanism, storage and one or more processors. The device records on the storage information including the acoustic signal, the information being analyzable to identify one or both of wheezing or coughing episodes.

US 6,019,732 A discloses a device for monitoring tidal volume in a subject, the device comprising a flexible substrate for attachment to an animal or human. Sensing means comprising a series of semi-conductive and conductive sensors are attached to the substrate and connectable into an electric circuit. In response to changes in the expansion and contraction of the lungs in the subject the device reversely changes its resistance and thus converts changes in tidal volume to a signal output.

US 2002/0072685 A1 discloses a method and apparatus for monitoring and/or quantitatively measuring a patient's respiration using a flexible piezoelectric film sensor. The apparatus includes a flexible substrate that can be attached to a patient's neck. The apparatus further includes the piezoelectric film which converts acoustical waves generated by the patient's respiration into electrical signals.

US 2011/0301485 A1 discloses data binning methods and systems for estimating a subject's respiratory airflow from a body sound signal detected by an acoustic sensor on the subject's body. The methods and systems operate in a configuration mode followed by a monitoring mode. In the configuration mode, a body sound signal and respiratory airflow are detected by an on-body acoustic sensor and a spirometer, respectively, over a common time period. Time-aligned body sound signal and respiratory airflow data points are then generated and correlated with each other and stored in a look-up table. Then, in the monitoring mode, the look-up table is accessed using subsequent body sound signal readings to provide respiratory airflow estimates without further need for a spirometer.

US 2012/0041279 A1 and WO 2006/043278 A1 relate to systems and methods for stationary use, in particular in a hospital, the systems not being portable or wearable. The systems comprise at least one sensor, in particular an electrode, which is placed on a subject's body and connected via leads to a device processing and analyzing the sensor measurements and thus monitoring, continuously or intermediately, the subject's respiratory status and health. When respiratory parameters reflect a loss in ventilation or other vital function, the systems and methods can maintain alarms or adjust operating parameters of a ventilator which is ventilating the subject.

Although devices that can be worn generally are known to enable comfortable use for a longer term, known solutions suffer from being disposable items, thus being wasteful and costly since sensors have to be replaced. On the other hand, traditional devices for similar purposes like stethoscopes or spirometers suffer from being less comfortable and not suitable for continuous 24 hour monitoring and/or long term use.

US 2015/0087922 A1 discloses a self-contained personal airflow sensing monitor. The wearable monitor includes two components, a flexible extended wear electrode patch and a removable reusable monitor recorder. The monitor recorder can be fitted during patient monitoring on the electrode patch and electrically connected thereto via contacts, and later removed for offloading of stored data or to receive revised programming. The monitor recorder stores data received from sensors of the patch in a memory and can be connected to a download station for retrieval of the stored data.

US 2013/ 0317333 A1 discloses a modular wearable sensor device. The wearable sensor comprises a first module having a first outer shell and a first printed circuit board within the first outer shell. The wearable sensor further comprises a second module coupled to the first module having a second outer shell and a second printed circuit board within the second outer shell. One of the first and second modules is disposable and the other of the first and second modules is reusable.

WO 2014/128090 A1 discloses a device for respiratory monitoring that has a disposable substrate for adhering to a patient torso and a re-usable electronics controller. The substrate comprises a body within which two elongate transducers are embedded for measuring deformation, the transducers being linked by conductors to the controller. The controller comprises a plastic housing and is mechanically joined to the substrate by use of an industrial grade hook and loop fastener.

US 2012/0190989 A1 relates to systems and methods that use wireless coupling of energy for operation of both external and internal devices including external sensor arrays and implantable devices. Such a device may be an external patch comprising a sensor array. An interrogator is positioned in proximity to the array to transmit energy to the ar-ray via an antenna.

US 2015/0126822 A1 relates to congestive heart failure risk status determination methods and related devices, wherein the parameters for determining the congestive heart failure risk status may comprise breath rate and breath volume. A monitoring system may comprise an adherent patch having sensors and an electronics layer encapsulated in an electronics housing. The electronic components, together with the electronics housing, may form a reusable electronics module separable from the patch component.

Although these devices can be worn for a longer period of time and comprise reusable parts, these solutions still suffer from being uncomfortable due to having rigid housings for the reusable parts. A further disadvantage is that the components have to be electronically connected via galvanic contacts, limiting reliability, reusability and ease of use and being less hygienic due to the direct contact.

An object of the present invention is to provide a system and a method being more efficient, resource saving, hygienic, and/or easier or more comfortable to use.

This object is achieved by a system according to claim 1 or by a method according to claim 14. Advantageous embodiments are subject of the dependent claims.

One aspect of the present invention relates to a system for facilitating detection of a respiratory status, wherein the system comprises a flexible patch being configured to be affixed or attached to a skin of a patient's body, in particular in the thoracic area and/or in the area of the respiratory tract. The patch comprises at least one sensor, preferably of a sensor arrangement, or at least a part of the sensor arrangement for sensing a body activity, in particular detecting a thoracic activity that preferably differs from tidal breathing and/or that corresponds to a respiratory status.

The sensor(s) or the sensor arrangement is/are configured to determine and/or output a sensor signal corresponding to said thoracic activity or said body activity. For that purpose, the sensors or the sensor arrangement can be configured to detect or sense the body activity related to the respiratory status when the patch is attached or affixed to the skin. Preferably, the system is configured to determine a and/or correlate to a respiratory airflow indicator based on the sensor signal.

The respiratory airflow indicator preferably depends on or corresponds or correlates to the airflow through the respiratory tract or system and/or to a volume and/or speed of air that circulates in the respiratory tract or system. In particular, the respiratory airflow indicator depends on, corresponds to, comprises or is a flow rate of air passing the respiratory tract or system. Alternatively or additionally, the respiratory airflow indicator depends on or corresponds or correlates to changes of properties of the airflow flowing through the respiratory system, and/or depends on relative changes of volume and/or speed of air that circulates in the respiratory tract or system.

The respiratory airflow indicator preferably corresponds to the respiratory status, i.e. to the properties of or depending on the respiratory tract, its physiology, geometry or tidal dynamics. Alternatively or additionally, the respiratory airflow indicator corresponds to or is configured to enable detection of changes of the respiratory status, i.e. changes to the properties of the respiratory tract, changes of the physiology of the respiratory tract, changes of the geometry of the respiratory tract and/or changes of the tidal dynamics of or within the respiratory tract.

Respiratory airflow indicators generally are known from or can be measured by spirometry. In particular, the respiratory airflow indicator reflects, comprises, or is formed by a so called flow-volume loop which is known to be generated from spirometry or corresponding data.

In the present disclosure, however, the respiratory airflow indicator is determined differently than the way spirometry does, namely with the sensor(s) or sensor arrangement of the wearable patch. The sensor signal for this purpose can be converted to the respiratory airflow indicator, preferably allowing the use of techniques well known from the analysis of spirometry results. The data collected through the use of the sensor(s) and/or the respiratory airflow indicator can be output and/or further analyzed with or without being output.

It is well known that a respiratory airflow rate is particularly suitable for assessing a respiratory status, in particular to identify obstruction or airflow limitation within the respiratory tract. However, such a respiratory airflow indicator that is determined by means of spirometry is not suitable for continuous monitoring or long term use nor allows wearing a measurement device. Wearable devices known in the art, however, make use of different approaches like recording and analyzing sounds directly which does not allow for sophisticated interpretation.

Thus, generating the respiratory airflow indicator can be an intermediate step between measurement by means of the sensor arrangement and analysis, in particular by a healthcare professional and/or by an (partly) automatic analysis system. This intermediate step, namely determination of the respiratory airflow indicator, comes along with advantages. The respiratory airflow indicator comprises additional information in comparison with the mere measurement results or sensor signal. In particular, determination of the respiratory airflow indicator adds information regarding anatomy or conditions of the respiratory tract that result in the measurement results - in particular by means of interpretation and/or correlation with anatomy-related references. Consequently, the respiratory airflow indicator enables a more reliable and comfortable determination of the respiratory status.

Respiratory airflow indicators or parts thereof can be determined from the sensor signal by means of comparison or correlation with reference signals like samples that assign a sensor signal fragment or data corresponding to a property of the sensor signal to the respiratory airflow indicator or a part of it. Such reference signals can, thus, replace the sensor signal in order to form and/or can be used to determine the respiratory airflow indicator.

In other words, advantages of tidal breathing measurement and wearable devices are combined in a synergistic manner since the sensor signal at least partially originating from the wearable patch is used to determine the respiratory airflow indicator in a first step and the respiratory airflow indicator in a second step can be more easily and reliably analyzed like results from tidal breathing measurement instruments, in particular although use of tidal breathing measurement like spirometers is avoided.

The present invention relates to a system for facilitating detection of a respiratory status, wherein the system comprises a flexible patch being configured to be attached or affixed to the patient's skin of a patient's body. The patch comprises at least one sensor, preferably of a sensor arrangement, for detecting or sensing a body activity of said patient's body when the patch is attached or affixed to the skin, and/or for detecting or sensing a thoracic activity, and/or corresponding to the respiratory status of the patient's body. The sensor or sensor arrangement is configured to determine and/or output a sensor signal, preferably corresponding to said body activity, in particular thoracic activity.

According to the invention, the system comprises a reusable add-on device being attachable to and/or holdable and/or detachable from the patch and being adapted to provide energy to and optionally to receive the sensor signal from the patch.

According to this aspect, it won't be necessary to dispose the complete wearable patch after use since the patch has two layers, one of them being the re-usable add-on device and the other one being configured to be directly attached and held on the skin. Thus, it is neither necessary to have sensor wires from a wearable patch to a measurement recorder or the like nor the measurement recorder or the like needs to form part of the patch and needs to be disposed after use.

This aspect of the present invention further provides the advantage that only such sensors that are or need to be directly in contact with the body will be part of the patch and other sensors or parts thereof without contact with the body and/or that do not need contact with the body can be part of the add-on device.

According to the invention, the add-on device is adapted to provide energy to the patch in a wireless manner.

Preferably, the patch and the add-on device are adapted to transmit and/or receive sensor signals wirelessly and/or without direct electrical (galvanic) contact. In particular, signal and/or energy transfer can be conducted inductively and/or capacitively. The patch and/or add-on device may comprise means for energy and/or signal transfer, such as electric coils and/or capacitors. Advantageously, direct electrical contact of the patch and the add-on device can be avoided providing a more reliable, reusable and/or hygienic system.

Preferably, the add-on device is patch-like and/or formed as a patch. In particular, the add-on device can be flexible and/or adherable. The add-on device preferably has its components arranged on or embedded in a (flexible) substrate, in particular without any additional housing or the like. Particularly preferably, the add-on device is formed at least essentially similar to the patch. In particular, components of the add-on device and of the patch can be arranged in a similar or corresponding manner. This advantageously increases wearing comfort and ease of use and/or is conducive to cost effective manufacturing of the proposed system.

Another further aspect of the present invention, which can be realized independently as well, relates to a method for facilitating detection of a respiratory status using the patch being configured to be or being attached or affixed to the skin of a patient's body. The patch comprises a sensor, preferably of a sensor arrangement, or at least a part of the sensor arrangement for sensing a body activity of said patient's body. The sensor or sensor arrangement is configured to determine and/or output a sensor signal corresponding to said body activity. Preferably, a respiratory airflow indicator is determined, preferably calculated, based on the sensor signal. Energy is provided to and optionally the sensor signal is received from the patch by the re-usable add-on device being attachable to and detachable from the patch. Thus, corresponding effects and advantages can be achieved.

A "patch" according to the present invention preferably is a flat device having a flat surface being configured for direct long term contact with skin. This surface comprises or is formed by an adhesive layer configured to adhere on skin. The adhesive layer preferably covers one side of the patch, preferably at least essentially or entirely, which is arranged to be in direct contact with the skin. However, there can be sections without adhesive layer as well. The patch preferably is flexible in a way that it follows the skin's shape and/or movement, and/or such that it can be torn in order to follow the skin's shape.

Preferably, the patch is flexible in lateral direction such that the patch can be stretched and/or compressed. Accordingly, the patch preferably is adapted to follow stretching of the skinwhere it is attached or attachable, affixed or affixable to. The patch can be water resistant or water tight, which, however, is not mandatory.

A patient's body according to the present invention preferably is the body of a human or an animal. The skin of a patient's body according to the present invention preferably is the body's surface, in particular an area of or adjacent to the respiratory or pulmonary system of the body.

A respiratory airflow indicator according to the present invention preferably is an indicator like or comprising one or more data values, curves, diagrams, tracts, functions, correlations, and/or assignments, and/or is, preferably directly, related to, representing or being indicative of airflow or airflow conditions in a respiratory tract.

In particular, the respiratory airflow indicator comprises or is formed by a respiratory airflow volume over time and/or a respiratory airflow rate, in particular over time or volume. The respiratory airflow indicator can comprise data being indicative of or corresponding to relative changes with or without reference or absolute values.

A respiratory airflow rate is a gauge for airflow volume per time, which can have the dimension liter/sec or mililiters/sec. The respiratory airflow volume over time, in the following referred to as transient respiratory airflow, can have the dimension mililiter and might be zero without breathing activity like at the point between breathing in and breathing out. At this point, of course, the respiratory airflow rate is zero as well.

Particularly preferably, the respiratory airflow indicator is or comprises a so-called flow-volume loop - which assigns or is an assignment of the respiratory airflow rate to the lung volume - or corresponding information / data.

Alternatively or additionally, the respiratory airflow indicator is, corresponds to or comprises data corresponding to or being indicative (in particular over time) of one or more of:
- TLC: Total lung capacity: the volume in the lungs at maximal inflation, the sum of VC and RV.
- TV: Tidal volume: that volume of air moved into or out of the lungs during quiet breathing (TV indicates a subdivision of the lung; when tidal volume is precisely measured, as in gas exchange calculation, the symbol TV or VT is used.)
- RV: Residual volume: the volume of air remaining in the lungs after a maximal exhalation
- ERV: Expiratory reserve volume: the maximal volume of air that can be exhaled from the end-expiratory position
- IRV: Inspiratory reserve volume: the maximal volume that can be inhaled from the end-inspiratory level
- IC: Inspiratory capacity: the sum of IRV and TV
- IVC: Inspiratory vital capacity: the maximum volume of air inhaled from the point of maximum expiration
- VC: Vital capacity: the volume of air breathed out after the deepest inhalation.
- VT: Tidal volume: that volume of air moved into or out of the lungs during quiet breathing (VT indicates a subdivision of the lung; when tidal volume is precisely measured, as in gas exchange calculation, the symbol TV or VT is used.)
- FRC: Functional residual capacity: the volume in the lungs at the endexpiratory position
- RV/TLC%: Residual volume expressed as percent of TLC
- VA: Alveolar gas volume
- VL: Actual volume of the lung including the volume of the conducting airway.
- FVC: Forced vital capacity: the determination of the vital capacity from a maximally forced expiratory effort
- FEVt: Forced expiratory volume (time): a generic term indicating the volume of air exhaled under forced conditions in the first t seconds
- FEV1: Volume that has been exhaled at the end of the first second of forced expiration
- FEFx: Forced expiratory flow related to some portion of the FVC curve; modifiers refer to amount of FVC already exhaled
- FEFmax: The maximum instantaneous flow achieved during a FVC maneuver
- FIF: Forced inspiratory flow: (Specific measurement of the forced inspiratory curve is denoted by nomenclature analogous to that for the forced expiratory curve. For example, maximum inspiratory flow is denoted FIFmax. Unless otherwise specified, volume qualifiers indicate the volume inspired from RV at the point of measurement.)
- PEF: Peak expiratory flow: The highest forced expiratory flow measured with a peak flow meter
- MVV: Maximal voluntary ventilation: volume of air expired in a specified period during repetitive maximal effort

Preferably, the lung volume or capacity, in particular FVC, can be input and/or used for determining the respiratory airflow indicator, in particular for correlation and/or determination and/or as an absolute reference for determination of the flow-volume loop.

Alternatively or additionally, the respiratory airflow indicator can be determined or is still valuable without the FVC absolute measurement, as the changes of the airflow per respiratory cycle and frequency correlates with the asthma progression, and the absolute number FVC enables only to fix the scale.

Thus, the respiratory airflow indicator can be improved by correlation, comparison or more generally using the FVC or a different parameter relating to an absolute volume or capacity of the lung for determination of the respiratory airflow indicator.

However, the respiratory airflow indicator alternatively or additionally can correlate to or indicate variations or changes without reference to or providing absolute values or references. That is, the respiratory airflow indicator can characterize or be indicative of relative changes only.

"Flow" or "airflow" according to the present invention preferably means flow of ambient air through the respiratory tract towards the lungs or in the opposite direction, wherein gas which is breathed in or out - covering exhalation air - is air according to the present invention and the corresponding flow is called airflow although its composition might differ at least regarding carbon dioxide and oxygen shares from ambient air. Thus, the terms "flow", "airflow", and "inhalation and/or exhalation air" are used synonymously and can be replaced by one another.

Further aspects, features and advantages of the present invention can be obtained from the claims, the figures and the following description of preferred embodiments referring to the figures. In the figures:
- Fig. 1: shows a schematic view of a system according to the present invention;
- Fig. 2: a schematic view of a patch according to the present invention;
- Fig. 3: a schematic cross-section of the patch according to cutting line III-III of Fig. 2;
- Fig. 4: a schematic view of an add-on device according to the present invention; and
- Fig. 5: a schematic flow chart of an exemplary method according to the present invention.

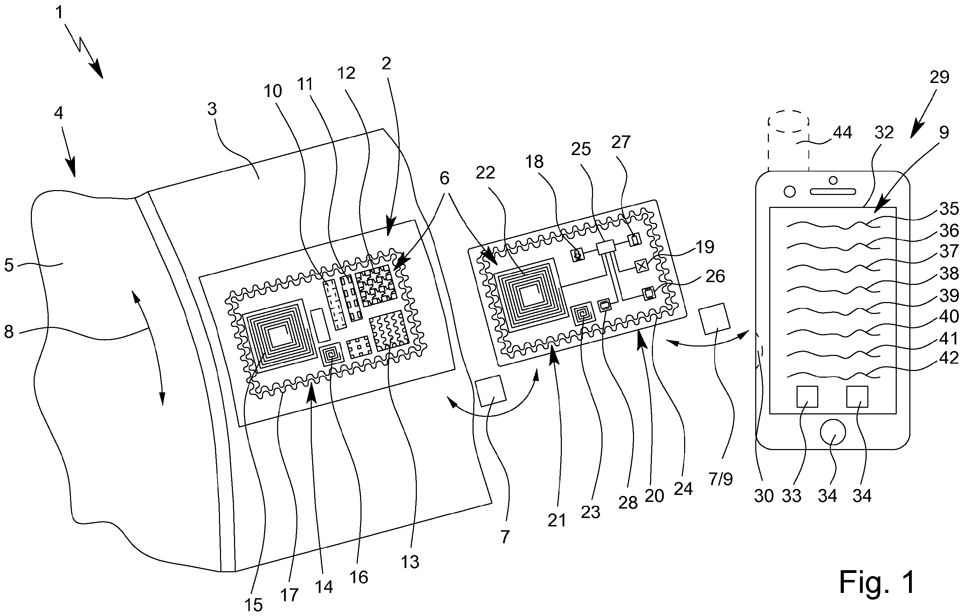

In Fig. 1 a system 1 for detecting a respiratory status according to the present invention is depicted schematically.

The system 1 comprises a flexible patch 2. This patch 2 is configured to be affixed or attached to a skin 3 of a patient's body 4, in particular a human or animal.

In the example shown in Fig. 1, the patch 2 is set or arranged in the range or area of a respiratory tract 5 of the body 4 such that the respiratory tract 5 or activity within or in connection with the respiratory tract 5 can act on the patch 2. In particular, the patch 2 is adapted to, arranged or arrangeable on the skin 3 facing away or opposite to the respiratory tract 5 with reference to the skin 3.

The system 1 comprises at least one sensor arrangement 6 for sensing a thoracic or body activity, preferably related to the respiratory status as defined in the introductory part of the description.

Preferably, the patch 2 comprises the sensor arrangement 6 or a part thereof. In particular, the sensor arrangement 6 is configured to sense the thoracic or body activity when the patch 2 is attached or affixed to or arranged at the skin 3.

The sensor arrangement 6 preferably is configured to determine and/or output a sensor signal 7 which corresponds to the body activity.

Preferably, the sensor arrangement 6 is configured to sense a body activity that depends on a respiratory status and/or a respiratory airflow 8 within the respiratory tract 5.

According to one aspect of the present invention, the system 1 is configured to determine, in particular calculate, a respiratory airflow indicator 9 based on the sensor signal 7. Using other words, the sensor signal 7 in the system 1 of the present invention is used to determine or calculate the respiratory airflow indicator 9.

Regarding the meaning of the term "respiratory airflow indicator 9" reference is made to the introductory part of the description. In particular, the respiratory airflow indicator 9 is or comprises one or more traces, vectors, functions, or different data, corresponding to the thoracic or body activity depending on the respiratory airflow 8 and, thus, preferably being indicative of the respiratory status.

The sensor signal 7 preferably is mathematically transformable into the respiratory airflow indicator 9. In particular, it is preferred that the sensor signal 7 comprises or is composed of data sensed by means of the sensor arrangement 6, wherein the data preferably corresponds to the respiratory airflow 8 or the effects that the respiratory airflow 8 causes while moving in the respiratory tract 5. That is, the sensor arrangement 6 preferably is adapted to measure or measures effects the respiratory airflow 8 inside the respiratory tract 5 causes. Accordingly, the respiratory airflow indicator 9 determined or calculated based on the sensor signal 7 preferably corresponds to effects the respiratory airflow 8 has within the respiratory tract 5. This enables detecting the status of the respiratory tract 5, also referred to as respiratory status, in particular either by a health care professional or automatically (assisted).

In the following paragraphs, the sensor arrangement 6 or parts thereof that are preferably comprised in the patch 2 are described in further detail.

The sensor arrangement 6 preferably comprises at least a stretch sensor 10 configured for sensing a stretch, in particular a degree of stretch, of the skin 3. The stretch sensor 10 preferably is arranged on, in, or by the patch 2. Due to the flexibility of the patch 2, the stretch sensor 10 can be stretched via the patch 2 when the skin 3 is stretched during breathing activity.

The degree of stretch detected preferably corresponds to the degree by which the skin 3 is stretched. Provided that the patch 2 is properly attached to the skin 3 in the thoracic area or area of the respiratory tract 5, i.e. on the breast of the body 4, stretch of the skin 3 correlates to the breathing movements and, thus, to the respiratory airflow 8 and its effects on the respiratory tract 5 can be detected by means of the stretch sensor 10. Accordingly, the stretch sensor 10 is configured to measure stretch of the skin 3 which depends on the breathing activity, to the respiratory airflow 8, or its effects on the respiratory tract 5.

The sensor signal 7 preferably represents the degree of stretch. Thus, the sensor signal 7 enables determination of the respiratory airflow indicator 9 based on information regarding the respiratory tract 5 and the respiratory airflow 8 on which the stretch or degree of stretch depends.

In particular, by breathing in and breathing out the skin 3 is stretched to various degrees. The deeper the breathing is, the higher the differences in stretching can be measured. Further, spasms related to the respiratory tract 5 can influence the stretch, in particular the maximum measureable stretch variation or amplitude. This can be used to determine the respiratory airflow indicator 9.

Alternatively or additionally, the stretch sensor 10 and/or the analysis of the sensor signal 7 corresponding to or comprising measurement results from the stretch sensor 10 can distinguish between different positions of stretch and/or directions of stretch.

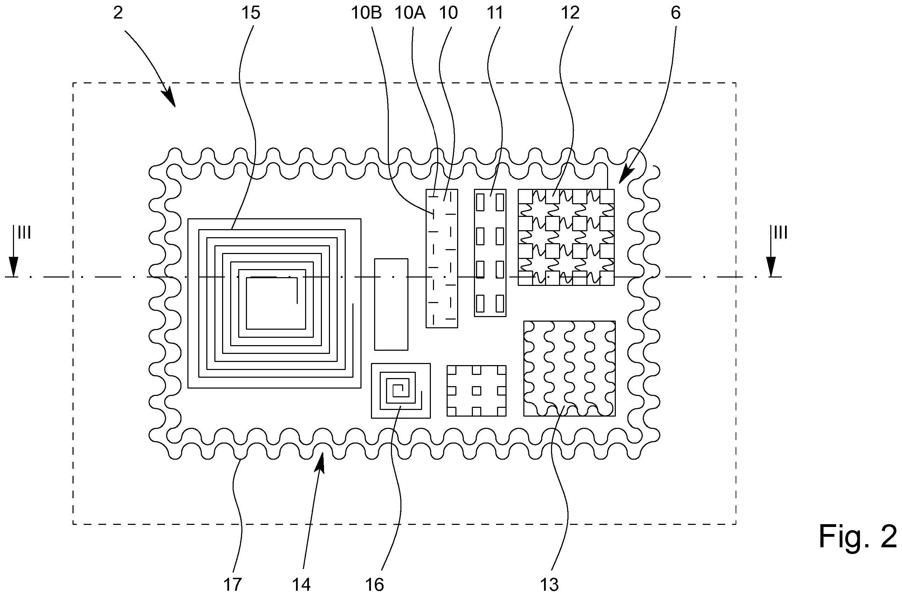

Fig. 2 shows an enlarged top view of patch 2. The stretch sensor 10 can comprise one or more strain sensor elements 10A, 10B. In the example shown, the strain sensor element 10A has a different orientation than a different strain sensor element 10B. In particular, the strain sensor elements have orientations which are at least essentially perpendicular to one another. The strain sensor elements 10A, 10B preferably are lengthy, in particular forming strain sensor stripes (each). However, different implementations may be possible and also in focus of the present invention.

By means of the stretch sensor 10, lengthwise stretching of the thorax area of body 4 and along a perimeter of the body 4 can be used separately or in combination for further interpretation. This can be used to determine or improve the respiratory airflow indicator 9. This can improve the determination of the respiratory airflow indicator 9 and, thus, of the respiratory status which can be found based on the respiratory airflow indicator 9.

Returning to Fig. 1, the system 1 or the sensor arrangement 6 optionally comprises one or more additional sensors. The sensor arrangement 6, in particular patch 2, preferably comprises a temperature sensor 11 by means of which the temperature of the skin 3 and/or of the ambient can be measured.

Alternatively or additionally, the system 1, patch 2 and/or sensor arrangement 6 comprises an ECG sensor 12 and/or an EMG sensor 13 for measuring heart activity signals and/or muscle activity signals, respectively.

In particular, the EMG sensor 13 can be used to detect muscle activities that occur during breathing and/or of or depending on the respiratory tract 5 in order to facilitate or improve the determination or calculation of the respiratory airflow indicator 9.

The temperature sensor 11 can be used to improve the results regarding determination of the respiratory airflow indicator 9 as well. In particular, the temperature measured by the temperature sensor 11 which preferably is arranged on the patch 2, can be used to ensure that the patch 2 is properly attached or affixed to the skin 3. For example, measuring a temperature different from the expected body surface temperature can be used as an indicator that the patch 2 is not properly attached to the skin 3. Accordingly, the temperature sensor 11 can be used for detecting handling errors, error handling or the like.

The ECG sensor 12 and/or the EMG sensor 13 and/or the temperature sensor 11 preferably are arranged on or form part of the patch 2 so that it can directly be influenced or affected, influenceable, or affectable by the (thoracic) body activity like a strain caused by stretching of the skin 3.

The patch 2 preferably further comprises a patch interface 14 for receiving and/or transmitting energy and/or information like the sensor signal 7, a part thereof, or other/further data.

The patch interface 14 in the example shown comprises a power coupling device 15, a data coupling device 16, and/or an antenna 17.

The power coupling device 15 preferably is configured for inductive and/or near field power transfer. Particularly preferably, the power coupling device 15 is configured for wireless energy transfer. Here, the power coupling device 15 comprises or is formed by a coil which can be used for inductive energy transfer. Additionally or alternatively, the power coupling device 15 can comprise different means for power transfer like galvanic contacts or means for capacitive energy transfer like a capacitor plate (not shown).

The data coupling device 16 can be a separate part of the power coupling device15 or can be part of the power coupling device 15 whereas the power coupling device 15 can be used for data coupling as well. In the example shown in Fig. 1 to 3, the data coupling device 16 is formed by or comprises a high frequency coil. This high frequency coil alternatively can be used to form a resonant circuit for operation of one or more of the sensors or a transmitter or receiver or transreceiver of the patch 2 and/or for to be used in the data and/or energy transfer.

The antenna 17 preferably is configured for wireless transmission of the sensor signal 7. Alternatively or additionally, further sensor information, status information regarding operation of the patch 2 or its components can be transferred by means of the antenna 17. However, such data transfer alternatively or additionally can be supported by the patch 2 in different (wireless) manner, for example by means of inductive coupling, e.g. via power coupling device 15 or data coupling device 16, or by means of galvanic contacts (not shown).

Wireless interfaces, e.g., a wireless power coupling device 15, and/or a wireless data coupling device 16, and/or a wireless antenna 17 is/are preferred since avoiding galvanic contacts can improve reliability and easy use, in particular in consideration of the field of application for the patch 2.

The patch 2 can furtherhave transistors and active or passive electronic components for supporting the detecting processes, energy transfer, energy (intermediate) storage which are not shown in detail. Such devices and circuits, however, are well known in the prior art like from sensor data sheets.

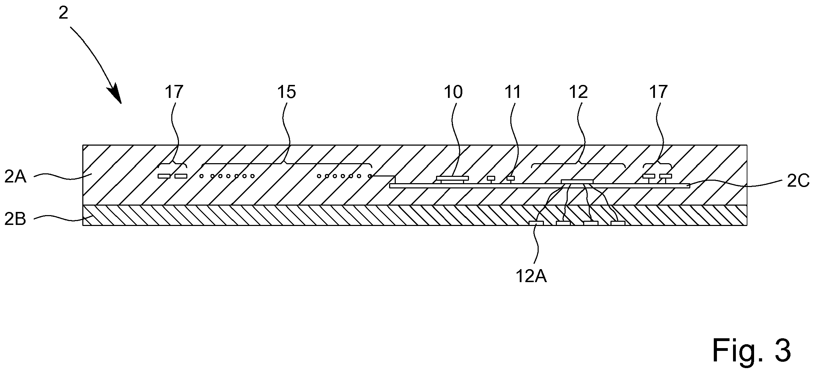

Fig. 3 shows a schematic cross section of the patch 2 according to the cutting line III - III of Fig. 2.

The patch 2 preferably comprises multiple layers 2A, 2B. A carrier layer 2A of patch 2 can cover or carry most of the electronic or sensoric elements of the patch 2. In particular, the stretch sensor 10, the temperature sensor 11, the EMG sensor 13, the patch interface 14, the power coupling device 15, the data coupling device 16, and/or the antenna 17 can be arranged in and/or on this carrier layer 2A, preferably being covered or enclosed in a water tight manner.

According to the invention, the patch 2 comprises an adhesive layer 2B which is configured to attach or affix the patch 2 to the skin 3. This adhesive layer 2B can cover one side of the carrier layer 2A, preferably in its entirely or at least essentially or partially, in particular at least at a peripheral area along the (entire) edge of the patch 2 or carrier layer 2A.

The adhesive layer 2B preferably overlaps at least the stretch sensor 10. This improves coupling of the stretch sensor 10 with the skin 3. Thus, the accuracy of the stretch sensor 10 and of the respiratory airflow indicator 9 calculated based on the measurement results from the stretch sensor 10 can be improved.

The patch 2 can comprise electrodes 12A, preferably for or of the ECG sensor 12, which can be arranged at or inside the surface of the adhesive layer 2B such that galvanic contact of the one or more of the electrodes 12A to the skin 3 is enabled. Thus, the adhesive layer 2B preferably does not cover the electrodes 12A. Alternatively, the adhesive layer 2B is (at least partially) electrically conductive as far as it covers electrodes 12A or forms the electrodes 12A.

The patch 2 can comprise one or more, preferably flexible, circuit boards 2C or printed circuit boards for connecting or interconnecting the sensors and further components of the patch 2.

A circuit board 2C can be arranged inside or on the carrier layer 2A. The circuit board 2C can be flexible and/or comprise conductors for connecting one or more of the sensors and/or components of the patch 2 like the patch interface 14, the power coupling device 15, the data coupling device 16, and/or the antenna 17. The circuit board 2C can be a printed circuit board and/or a laminated circuit board.

The sensor arrangement 6 preferably comprises a sonic sensor 18. The sonic sensor 18 is configured for detecting sound or vibrations caused by the respiratory airflow 8, activity of the respiratory tract 5 and/or sound or vibrations caused by the respiratory airflow 8 acting on or resulting from interaction with the respiratory tract 5.

The sonic sensor 18 can comprise or be formed by a microphone and/or structure-borne sound sensor. Detecting structure-borne sound is particularly preferred since this is an easy and reliable measure to gather data which is correlated to and allowing determination, calculation and/or improvement of the respiratory airflow indicator 9.

Alternatively or additionally, the sensor arrangement 6 comprises a movement sensor 19 for detecting movement of the body 4, in particular movement of the body's skin 3. The movement sensor 19 can be adapted to sense movement like lifting and lowering of the ribcage or the chest as caused by breathing activity.

The movement sensor 19 can be used to detect a breath frequency and breathing phases. This enables or simplifies interpretation of further sensor information and of the vital status of the body 4.

The movement sensor 19 can also be used for measuring the pulse. Alternatively or additionally, the sensor arrangement 6 can comprise a separate pulse sensor or a different one of the sensors of sensor arrangement 6 can be used to sense the pulse of nearby arteries.

The sensor signal 7 preferably comprises or transfers data or measurement results from the sensor arrangement 6, in particular from one or more of the stretch sensor 10, the temperature sensor 11, the ECG sensor 12, the EMG sensor 13, the sonic sensor 18, and/or the movement sensor 19. In this regard, it is preferred that the sensor signal 7 represents the degree of stretch, the temperature, the ECG-values, the EMG-values, the sound caused by the respiratory airflow 8, and/or the movement of the body 4.

The system 1 is preferably configured to determine or to calculate the respiratory airflow indicator 9 by analyzing stretch, temperature, ECG-value, EMG-value, sound, and/or movement, preferably of the thorax.

According to the present invention, the system 1 comprises at least two parts being connectable to and/or separable from one another.

The system comprises an add-on device 20. The add-on device 20 is attachable to and detachable from the patch 2. Thus, the patch 2 can comprise the detachable and/or re-attachable add-on device 20. In particular, the add-on device 20 can be or form a layer of the patch 2 when the system 1 is ready for use.

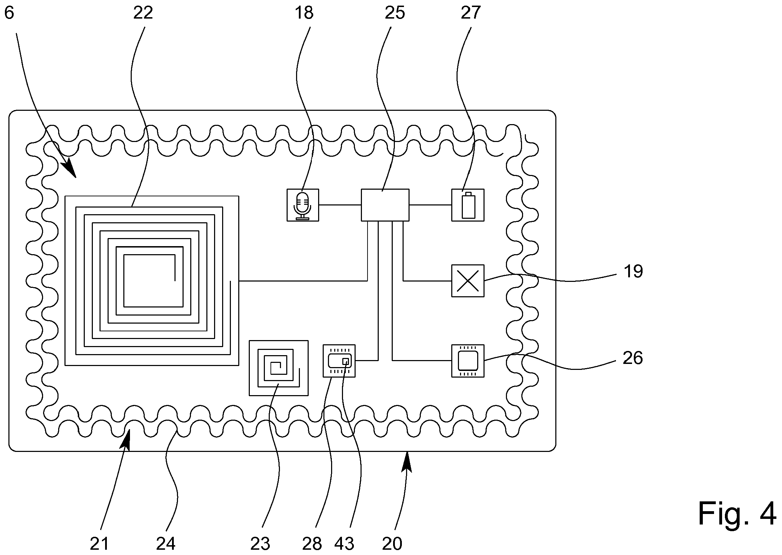

In Fig. 4 a top view of the add-on device 20 is depicted. The add-on device 20 doesn't need to be flexible, it may be (at least essentially) rigid or dimensionally stable. The add-on device 20 can comprise a circuit board, preferably a printed circuit board, on which contacts can be connected to components of the add-on device 20 and/or formed by them, like in the present case where the coil forms the power coupling device 22 of the add-on device 20.

The add-on device 20 is reusable while the patch 2, which is used in direct contact with the skin 3, preferably is a disposable device to be used only once due to hygienic reasons.

The add-on device 20 is configured to provide energy to the patch 2. Alternatively or additionally, the add-on device 20 is configured to receive the sensor signal 7 - entirely or parts of it - from the patch 2.

The add-on device 20 is re-useable. For this purpose, the add-on device 20 can be attached to and/or coupled with multiple patches 2, preferably successively.

The system 1 preferably is configured such that a mechanical, electrical, and/or data connection between the add-on device 20 and the patch 2 can be created if or by attaching the add-on device 20 to the patch 2 or connecting or mounting the add-on device 20 on the patch 2. Particularly preferably, such connection is (automatically) created when the add-on device 20 is connected to or mounted on the patch 2. Alternatively or additionally, said connection preferably is (automatically) disconnected when the add-on device 20 is separated or removed from the patch 2. This facilitates very easy operation.

Preferably, the add-on device 20 is patch-like and/or formed as a patch. In particular, the add-on device 20 may comprise a preferably flexible carrier layer and/or an adhesive layer with its components being located on or embedded in the carrier layer.

Preferably, the add-on device 20 is at least essentially adapted or formed similar to the patch 2. The add-on device 20 preferably has the same or similar dimensions as the patch 2. Components of the add-on device 20 are preferably arranged in a manner similar or corresponding to the arrangement of components on the patch 2. Particularly preferably, components of the add-on device 20 which are identical or similar or correspond to components of the patch 2, such as the power coupling device 22, are located at least essentially in a similar spot as the corresponding components on patch 2.

The system 1 preferably is a multi-part modular system 1, whereas the patch 2 forms a first module and the add-on device 20 forms a second module, the modules being configured to be combined for forming a functional system 1 or part thereof. Thus, the patch 2 can comprise at least two parts, namely the carrier layer 2A and/or adhesive layer 2B on the one hand, and the add-on device 20 on the other hand. These two parts together form the patch 2, system 1 or part of the system 1. These parts taken together preferably comprise the sensor arrangement 6. The sensor arrangement 6 can comprise or be formed by components of both of the patch 2 and the add-on device 20.

The sensor arrangement 6 preferably is provided on or covers both of the patch 2 and the add-on device 20. This allows to reuse sensors and further components of the system 1 which do not need to have direct contact or proximity to or needs to be influenced by the skin 3, body 4 and, thus, can form part of the add-on device 20.

Preferably, at least the sonic sensor 18 and/or the movement sensor 19 are arranged on the add-on device 20 rather than on the patch 2.

In Fig. 1, the add-on device 20 is shown moved away from the patch 2. However, during operation it is preferred that the add-on device 20 is attached to the patch 2 such that the add-on device 20 covers or overlaps the patch 2.

The add-on device 20 and the patch 2 are preferably configured to be connected. In particular, the add-on device 20 has connecting means to affix it to the patch 2 like glue, adhesive film, tape, or other means for, preferably detachably, holding the add-on device 20 at and/or on the patch 2.

The add-on device 20 together with the patch 2 preferably form a piggyback configuration, where the patch 2 is connected or connectable to the skin 3 and where the add-on device 20 is connected or connectable to the patch 2 on a side of the patch 2 facing away from the skin 3 or from the side of patch 2 being arranged to be affixed to the skin 3.

The add-on device 20 in the example shown comprises an add-on device interface 21 configured for connecting the add-on device 20 to the patch 2 electrically and/or electronically. The add-on device 20, in particular the add-on device interface 21, can comprise a power coupling device 22, a data coupling device 23, and/or an antenna 24.

The power coupling device 22 of the add-on device 20 preferably corresponds to the power-coupling device 15 of the patch 2. The power coupling devices 15, 22 can be configured and arranged in such a way that power coupling from the add-on device 20 to the patch 2 is enabled. In particular, both power coupling devices 15, 22 work inductively, comprise or are formed of one or more coils and/or comprise capacitive coupling means like one or more capacitive plates or different means for energy transfer.

Arranging the power coupling devices 15, 22 adjacent to or overlapping one another, thus, establishes a power transfer connection which allows to transfer power, in particular current, between the add-on device 20 and the patch 2. Thus, the add-on device 20 is configured to provide electrical energy to the patch 2 wirelessly or contactlessly. Additionally galvanic contacts might be provided or the power coupling devices 15, 22 might also comprise or be realized by contacts for establishing an electric connection between the patch 2 and add-on device 20.

Alternatively or additionally, the add-on device 20 is configured to receive the sensor signal 7 or parts thereof from the patch 2, preferably in a wireless manner. This can be achieved using the data coupling device 23 of the add-on device 20, which preferably corresponds to the data coupling device 16 of the patch 2.

The data coupling devices 16, 23 can be coupled and used for establishing a data connection by arranging them close to one another. In the example depicted in Fig. 1, the data coupling devices 16, 23 work inductively or are realized by coils. Alternatively or additionally, a capacitive or different coupling can be enabled by the data coupling devices 16, 23.

The data coupling devices 16, 23 are preferably configured to enable transfer of data, in particular of the sensor signal 7 or a part of the sensor signal 7. Thus, the sensor signal 7 or components thereof can be transmitted from the patch 2 to the add-on device 20. Thus, measurement data obtained by the stretch sensor 10 and, optionally, measurement data from the temperature sensor 11, the ECG sensor 12, the EMG sensor 13, and/or further sensors that might be comprised in or realized by the patch 2 can be forwarded to the add-on device 20.

In one alternative configuration, the power coupling devices 15, 22 can be used for data transfer. In just another alternative, the antenna 24 of the add-on device 20 can be used for data transfer between the patch 2 and the add-on device 20. However, it is preferred that the antenna 17 of the patch 2 or the antenna 24 of the add-on device 20 can be used for forwarding the sensor signal 7, the respiratory airflow indicator 9 or further information to remote devices. Thus, it is not mandatory that both the patch 2 and the add-on device 20 need to have an antenna 17, 24. It might be sufficient only one of them has an antenna 17, 24.

The power coupling devices 15, 22 and/or data coupling devices 16, 23 preferably are configured to be arranged (completely) overlapping in order to facilitate high coupling factors. In particular, the power coupling devices 15, 22 are similar in dimension and/or construction. Alternatively or additionally, the data coupling devices 16, 23 are similar in dimension and/or construction.

Further, it is preferred that the add-on device 20 can be placed on patch 2 such that the power coupling devices 15, 22 are overlapping, preferably in a manner that a focus or center of the respective power coupling devices 15, 22 are arranged on or cut by an axis which is at least basically perpendicular to both of the power coupling devices 15, 22.

Thus, provided that the power coupling devices 15, 22 are coils, like in the example shown, the coils are similar, placed adjacent to one another, and/or placed at in least essentially parallel planes, surfaces or layers such that coupling between them is enabled. The same preferably applies to the data coupling devices 16, 23.

In a further alternative, the sensor signal 7 or its components are transmitted or transmittable to the patch 2 and the patch 2, preferably using the antenna 17, can transmit the sensor signal 7 to a remote device.

The sensor signal 7 as transmitted to a remote device can comprise measurement results from both the add-on device 20 and the patch 2.

Conductors like wires used on the patch 2, for example to connect or interconnect components like the stretch sensor 10, temperature sensor 11, ECG sensor 12, EMG sensor 13, patch interface 14, power coupling device 15, data coupling device 16, and/or antenna 17, preferably are meandering, following a wavy path, and/or more generally are configured to be reversibly deformed or stretched allowing the patch 2 to be stretched while damage of conductor lines or wires is avoided.

In the example shown, conducting lines forming antenna 17 have a structure that allows reversible stretching without damage. Further conductors like those forming the coils of the power coupling device 15 or the data coupling device 16 can be constructed accordingly (not shown).

It is preferred that the structure of the conductors at least allow stretching the patch 2 about 20%, preferably 30%, without the integrity of the conductors being affected. Preferably, enough surplus length of the conductor lines is provided to enable stretching the patch 2 without affecting the conductor lines. In particular, the distance bridged by multiple - preferably more than five - meanders, periods, waves or a different (repetitive) structure in order to allow stretching preferably is less than 80%, 70% or 60% of the track length of the conductor line.

The conductor lines used within the add-on device 20 do not necessarily need to form wavy tracks as well. However, this is possible and might be reasonable to enhance coupling factors. However, conductor lines used in the add-on device 20 can be at least basically straight in order to avoid potential negative effects or increased effort in production. In particular, lines being no part of the power coupling device 22, the data coupling device 23, and/or antenna 24 should be at least basically straight.

The add-on device interface 21 preferably is configured for transmitting and/or receiving the sensor signal 7, and/or the respiratory airflow indicator 9. In particular, the add-on device 20 is adapted to send via the add-on device interface 21 the sensor signal 7 and/or the respiratory airflow indicator 9 to a remote device.

Alternatively or additionally, a warning 41 and/or instructions 42 can be transmitted or sent. In this regard, it is preferred that the add-on device 20 or the add-on device interface 21 is configured for transmitting and/or receiving warnings 41 and/or instructions 42 in a wireless manner. However, different solutions might be possible alternatively or additionally.

The add-on device 20 preferably comprises further components for control, detection, determination, and/or calculation of the respiratory airflow indicator 9.

The system 1, preferably the add-on device 20, in a further aspect of the present invention comprises a processing device 26 which is configured to analyze the sensor signal 7. In the example shown, the processing device 26 forms part of the add-on device 20. However, alternatively or additionally, the processing device 26 can be arranged on or form part of the patch 2 or a different device of system 1 as well.

The add-on device 20 preferably comprises a memory 28 for storing or intermediate storing of the sensor signal 7, parts thereof or corresponding data.

Alternatively or additionally, the add-on device 20 can be configured to store the respiratory airflow indicator 9 in the memory 28 after the respiratory airflow indicator 9 has been calculated from the sensor signal 7, preferably by means of the processing device 26.

However, it is not mandatory that the respiratory airflow indicator 9 is stored after it has been calculated based on the sensor signal 7. For example, it can be forwarded or transmitted directly or the like. Alternatively or additionally, the respiratory airflow indicator 9 can be directly used to determine a different gauge corresponding to the respiratory status or to determine the respiratory status.

For calculating the respiratory airflow indicator 9 by the processing device 26 based on the sensor signal 7, the sensor signal 7 can be correlated with one or more reference signals 43 or values which can be stored in memory 28. Alternatively, the add-on device 20 can forward the sensor signal 7, and the calculation of the respiratory airflow indicator 9 is conducted externally.

In a further aspect of the present invention, the processing device 26 is configured for determination or calculation of a respiratory airflow limitation indicator 40 or a different irregularity relating to the respiratory tract 5 based on the respiratory airflow indicator 9 or the sensor signal 7. This can enable deeper analysis by a user or guiding a user regarding a diagnosis or treatment without or accompanied by automatically providing a diagnosis or treatment instructions. The processing device 26 can be configured to generate the warning 41 and/or instructions 42 if the respiratory airflow limitation indicator 40 is detected or exceeds a threshold.

The add-on device 20 preferably has a control device 25 for controlling functions of the add-on device 20 and/or of the patch 2. In particular, the control device 25 is configured for controlling transfer of energy to the patch 2. In this regard, the control device 25 can receive energy from an energy storing means 27 which can be comprised in or on the add-on device 20. Energy from the energy storing means 27 can be forwarded to the patch 2 via the power coupling devices 15, 22.

It is preferred that the control device 25 generates an alternating current which is provided to the power coupling device 22 of the add-on device 20. With this alternating current, the power coupling device 22 can generate an alternating electrical, magnetic or electromagnetic field. In the example shown, the alternating magnetic field is generated by the alternating current flowing through the coil windings. This alternating field in the power coupling device 15 of patch 2 induces or generates a current or voltage which can be used to source the stretch sensor 10 and/or further devices of patch 2.

The control device 25 alternatively or additionally can control the sensors of the add-on device 20 and/or of the patch 2 to measure and/or to transmit measurement results, preferably forming the sensor signal 7 or part of it.

The sensor signal 7 can comprise measurement results from various of the sensors but does not need to cover or take into account each of them. The sensor signal 7 can have multiple parts, components or can be different at different locations. In particular, the sensor signal 7 as transmitted or transmittable from the patch 2 to the add-on device 20 might cover measurement results from the sensors comprised in or on the patch 2 while the sensor signal 7 as optionally provided or transmitted by the add-on device 20 can cover in addition sensor results of sensors comprised in or on the add-on device 20.

In a further aspect of the present invention, the system 1 comprises at least one user device 29, which can realize or form the external device and/or can be realized or formed by or comprise a smartphone, tablet, laptop or different, preferably mobile and/or wirelessly connectable, device. The user device 29 preferably comprises a user device interface 30 for communication with the patch 2 and/or the add-on device 20. The user device 29 preferably comprises a user interface 31 like a display 32 and/or a control element 33 which can be part of or provided in addition to an input device 34.

Preferably, the sensor signal 7, the respiratory airflow indicator 9, or further information derivable from the sensor signal 7 and/or the respiratory airflow indicator 9 are transmitted or transmittable - preferably in a wireless manner - to the user device 29.

The user device 29 can, using the user interface 31, output at least the respiratory airflow indicator 9.

Alternatively or additionally, the user device 29 via the user interface 31 can output a transient respiratory airflow 35 (which is the airflow volume flowing to the respiratory tract 5 over time).

Alternatively or additionally, the user interface 31 can output a respiratory airflow rate 36 (which is the airflow volume over time which in a diagram can be displayed as the function of volume, preferably lung volume or respiration volume 39, in particular forming a so called flow-volume loop).

Alternatively or additionally, the warning 41 or instructions 42 can be output. The warning 41 or instructions 42 can be calculated based on the respiratory airflow indicator 9.

In the following, the respiratory airflow indicator 9, the transient respiratory airflow 35, the respiratory airflow rate 36, the inspiration time 37, the expiration time 38, the respiration volume 39, the warning 41, and/or the instructions 42 are referred to as analysis results. Thus, such analysis results can be provided to a user like a health care professional preferably by means of the user device 29.

The user device 29 preferably is configured to calculate or calculates such one or more analysis results from the sensor signal 7 and/or the respiratory airflow indicator 9 as provided by the add-on device 20 and/or the patch 2.

Alternatively or additionally, the processing device 26, which preferably is part of the add-on device 20 but can also be part of patch 2, can calculate and/or provide the analysis results at least partially. Then, the analysis can be completed by the user device 29 and output by the user interface 31.

The respiratory airflow indicator 9 alternatively or additionally can be realized by one or more of the analysis results. Thus, the respiratory airflow indicator 9 can be or correspond to at least one or more of a transient respiratory airflow 35, a respiratory airflow rate 36, an inspiration time 37, an expiration time 38, and a respiration volume 39.

The system 1 can be configured to automatically provide and/or automatically adapt treatment measures for treating the airflow limitation or different irregularity which is detectable based on the respiratory airflow indicator 9. For example, the user device 29 can comprise an inhalation device 44 or an inhalation device 44 can be realized independently of the user device 29, nevertheless forming part of the present system 1.

A further aspect of the present invention, which can be realized independently as well, relates to a method for detecting a respiratory status using the system 1 or patch 2, whereas the respiratory airflow indicator 9 is determined based on the sensor signal 7. Alternatively or additionally, energy is provided by and/or the sensor signal 7 is received from the re-usable add-on device 20 which is attachable to and detachable from the patch 2.

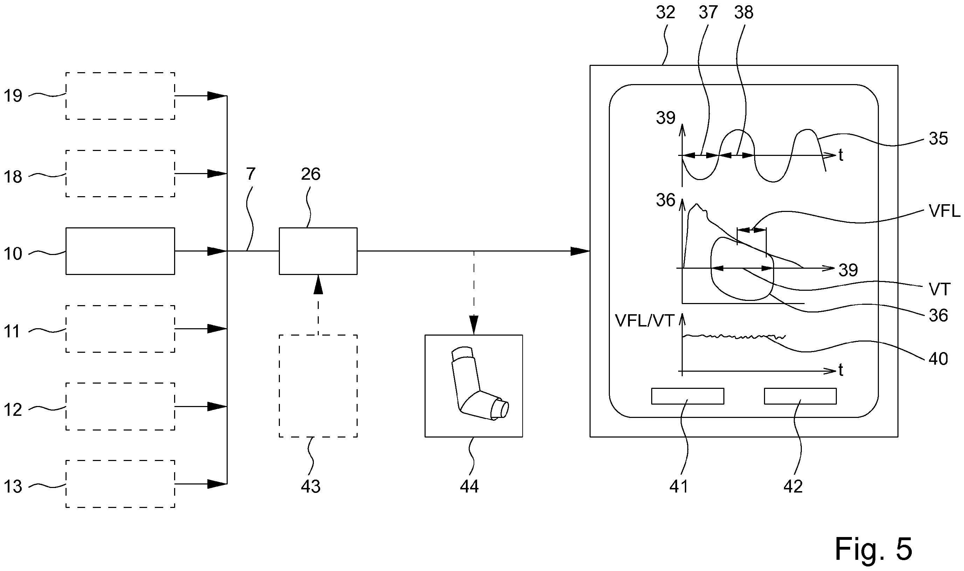

Fig. 5 shows a schematic flow chart of the method according to the present invention.

As depicted in Fig. 5, the sensor signal 7 preferably at least comprises measurement results from stretch sensor 10. Additionally or alternatively, the sensor signal 7 comprises measurement results from at least one or more of temperature sensor 11, ECG sensor 12, EMG sensor 13, sonic sensor 18, and movement sensor 19. However, it is not ruled out that further or different sensors are used and corresponding measurement results form part of the sensor signal 7.

The sensor signal 7 is provided to the processing device 26 which preferably is arranged on or forms part of the add-on device 20, but also can be realized by or form part of the patch 2 or a different device like the user device 29.

The processing device 26 preferably receives or the system 1 is configured to provide to the processing device 26 one or more reference signals 43. Those reference signals 43 can be stored in memory 28.

The one or more reference signals 43 preferably correspond to or relate to the sensor signal 7 or parts thereof. In particular, the reference signal(s) 43 can comprise one or multiple samples of potential sensor signal components. The reference signal(s) 43 can comprise a trace, graph, value or function relating to or forming a sample of a potential sensor signal 7 or part of a potential sensor signal 7. Thus, the reference signal 43 can be compared to or correlated with the sensor signal 7 or part of the sensor signal 7.

In addition, the reference signal 43 can be configured to assign the sensor signal 7 or components thereof to a respiratory airflow indicator 9 or components thereof. Accordingly, using the reference signal 43, the sensor signal 7 can be analyzed and the respiratory airflow indicator 9 can be determined or calculated.

In particular, the reference signal(s) 43 can be used for comparison with the sensor signal 7 in order to assign the sensor signal 7 to or identify a particular respiratory airflow indicator 9. This can be used to calculate - preferably by correlation - the respiratory airflow indicator 9.

The respiratory airflow indicator 9 preferably can be depicted by means of user interface 31 or display 32 in order to facilitate analysis of the respiratory airflow indicator 9 by means of a health care professional or there like. As shown in Fig. 5, one or more of the analysis results can be output.

Alternatively or additionally, the inhalation device 44 can be controlled based on the respiratory airflow indicator 9. In particular, the respiratory airflow indicator 9 can be used to determine a dose for medication and the inhalation device 44 can be controlled in accordance therewith such that a dose can be administered to the patient whose body 4 is monitored by means of the system 1 according to the present invention.

A particularly preferred output diagram is depicted on display 32 of Fig. 5 showing the respiratory airflow rate 36 depending on the respiration volume 39, also known as flow-volume loop since such output diagram shows a closed loop representing properties of the respiratory airflow 8. The flow-volume loop does not necessarily be depicted, but alternatively or additionally corresponding data can be analyzed.

By means of this diagram or corresponding data, an airflow limitation indicator 40 can be derived, where the airflow limitation indicator 40 preferably equals a limited volume VFL divided by the breathing volume VT. This airflow limitation indicator 40 can be calculated based on the respiratory airflow indicator 9, in particular by means of a graphical analysis based on a diagram or calculation based on corresponding information or data, even if the respiratory airflow rate 36 or respiratory airflow indicator 9 is not depicted.

Preferably, the airflow limitation indicator 40 can be derived even if no absolute values and/or only relative values or changes of respiratory airflow are available. This is in particular the case if no forced vital capacity FVC has been determined. In this case, the limited volume VFL and the breathing volume VT are only relative values, which, however, is irrelevant when determining their ratio, said ratio or airflow limitation indicator 40 thus still being indicative of any irregularities in the respiratory airflow.

The airflow rate limitation indicator 40 can be compared with a reference value or threshold in order to identify problems. If a problem is identified or if the threshold is reached or exceeded, the warning 41 and/or instructions 42 can be generated or output, preferably either to visit a health care professional or to administer a medicament for treating airflow limitation.

Although the present invention has been discussed referring to specific embodiments, the present invention shall not be limited to them. Further, it shall be emphasized that different aspects of the present invention can be combined in a different manner, wherein the same or different advantages can be obtained.

In particular, it is not mandatory to realize the modular system 1 together with aspects concerning calculation of the respiratory airflow indicator 9. Further, there are many ways to determine the respiratory airflow indicator 9 and, thus, a specific way does not need be realized in combination with aspects concerning the add-on device 20. However, the combination of those aspects is particularly advantageous although the aspects taken alone already provide advantages as well.

### Reference signs:

- 1: System
- 2: Patch
- 2A: Carrier layer
- 2B: Adhesive layer
- 2C: Circuit board
- 3: Skin
- 4: Body
- 5: Respiratory tract
- 6: Sensor arrangement
- 7: Sensor signal
- 8: Respiratory airflow
- 9: Respiratory airflow indicator
- 10: Stretch sensor
- 10A: Strain sensor element
- 10B: Strain sensor element
- 11: Temperature sensor
- 12: ECG sensor
- 12A: Electrode
- 13: EMG sensor
- 14: Patch interface
- 15: Power coupling device
- 16: Data coupling device
- 17: Antenna
- 18: Sonic sensor
- 19: Movement sensor
- 20: Add-on device
- 21: Add-on device interface
- 22: Power coupling device
- 23: Data coupling device
- 24: Antenna
- 25: Control device
- 26: Processing device
- 27: Energy storage means
- 28: Memory
- 29: User device
- 30: User device interface
- 31: User interface
- 32: Display
- 33: Control element
- 34: Input device
- 35: Transient respiratory airflow
- 36: Respiratory airflow rate
- 37: Inspiration time
- 38: Expiration time
- 39: Respiration volume
- 40: Airflow limitation indicator
- 41: Warning
- 42: Instructions
- 43: Reference signal
- 44: Inhalation device

## Claims

1. System (1) for facilitating detection of a respiratory status, wherein the system (1) comprises a flexible patch (2) being configured to be affixed or attached to a skin (3) of a patient's body (4) by means of an adhesive layer (2B), wherein the patch (2) comprises at least one sensor (10, 11, 12, 13), preferably of a sensor arrangement (6), for sensing a body activity corresponding to the respiratory status of said patient's body (4), and wherein the sensor (10, 11, 12, 13) or the sensor arrangement (6) is configured to determine and/or output a sensor signal (7) corresponding to said body (4) activity,
the system (1) comprising a re-usable add-on device (20) being attachable to and detachable from the patch (2), and
the add-on device (20) being adapted to provide energy to the patch (20) in a wireless manner.

2. System (1) according to claim 1, **characterized in that** the add-on device (20) comprises a flexible carrier layer with its components being located on or embedded in the carrier layer, and/or **in that** the add-on device (20) is adapted to receive the sensor signal (7) or parts thereof from the patch (2).

3. System (1) according to claim 1 or 2, **characterized in that** the sensor (10, 11, 12, 13) is or the sensor arrangement (6) comprises a stretch sensor (10) for sensing a stretch of the skin (3), preferably wherein the sensor signal (7) represents the degree of stretch, and/or **in that** the system (1) comprises a sonic sensor (18) configured for detecting sound caused by respiratory airflow (8) and/or a movement sensor (19) for detecting movement of the body (4), in particular of the body's skin (3), preferably wherein the sensor signal (7) represents the sound and/or movement.

4. System (1) according to claim 3, **characterized in** the system (1) is configured to determine or calculate a respiratory airflow indicator (9) by analyzing the stretch of the body (4), the sound caused by respiratory airflow (8), and/or the movement of the body (4).

5. System (1) according to any one of the preceding claims, **characterized in that** a mechanical, electrical, and/or data connection between the add-on device (20) and the patch (2) is created when the add-on device (20) is connected to the patch (2) and disconnected when the add-on device (20) is separated from the patch (2).

6. System (1) according to any one of the preceding claims, **characterized in that** the add-on device (20) is configured to receive the sensor signal (7) from the patch (2) in a wireless manner.

7. System (1) according to any one of the preceding claims, **characterized in that** the system (1) comprises a processing device (26) being arranged and configured for calculating a respiratory airflow indicator (9) based on the sensor signal (7), preferably by correlation of the sensor signal (7) with one or more reference signals (43) or values, preferably wherein the processing device (26) is arranged on or forms part of the patch (2) and/or add-on device (20).

8. System (1) according to claim 7, **characterized in that** the processing device (26) is configured for determination or calculation of a respiratory airflow limitation, a change thereof, or a different irregularity based on the respiratory airflow indicator (9), preferably wherein the processing device (26) is further configured to generate a warning (41) and/or instructions (42) if the respiratory airflow limitation is detected or exceeds a threshold.

9. System (1) according to any one of the preceding claims, **characterized in that** the system (1) comprises at least one user device (29), wherein the sensor signal (7), a respiratory airflow indicator (9), a warning (41), and/or instructions (42) are transmittable - preferably in a wireless manner - to the user device (29); and/or wherein the user device (29) comprises a user interface (31) which is configured to output a respiratory airflow indicator (9), a warning (41), and/or instructions (42) by means of this user interface (31).

10. System (1) according to any one of the preceding claims, **characterized in that** the add-on device (20) is formed as a flexible patch and/or is at least essentially adapted or formed similar to the patch (2).

11. System (1) according to any one of the preceding claims, **characterized in that** the patch (2) comprises a patch interface (14) for receiving and/or transmitting energy and/or information, in particular a power coupling device (15), a data coupling device (16) and/or an antenna (17).

12. System (1) according to any one of the preceding claims, **characterized in that** the add-on device (20) comprises an add-on device interface (21) configured for transmitting and/or receiving the sensor signal (7), a respiratory airflow indicator (9), a warning (41), and/or instructions (42), preferably in a wireless manner, and/or for connecting the add-on device (20) to the patch (2) electrically and/or electronically, in particular a power coupling device (22), a data coupling device (23) and/or an antenna (24).

13. System (1) according to claim 11 or 12, **characterized in that** the power coupling device(s) (15, 22) and/or data coupling device(s) (16,23) is/are configured for inductive, capacitive and/or near field power transfer.

14. Method for facilitating detection of a respiratory status using a patch (2) being configured to be affixed or attached to skin (3) of a patient's body (4) by means of an adhesive layer (2B), wherein the patch (2) comprises at least one sensor (10, 11, 12, 13), preferably of a sensor arrangement (6), for sensing a body activity corresponding to the respiratory status of said patient's body (4) and wherein the sensor (10, 11, 12, 13) or the sensor arrangement (6) is configured to determine and/or output a sensor signal (7) corresponding to said body (4) activity, and
using a re-usable add-on device (20) being attachable to and detachable from the patch (2),
wherein energy is provided to the patch (2) by the add-on device (20) in a wireless manner.

## Patentansprüche

1. System (1) zur Ermöglichung der Erfassung eines Atmungszustandes, wobei das System (1) ein flexibles Pflaster (2) aufweist, das so konfiguriert ist, dass es mittels einer Klebeschicht (2B) an einer Haut (3) eines Patientenkörpers (4) befestigt oder angebracht werden kann, wobei das Pflaster (2) mindestens einen Sensor (10, 11, 12, 13) aufweist, wobei das Pflaster (2) mindestens einen Sensor (10, 11, 12, 13), vorzugsweise einer Sensoranordnung (6), zum Erfassen einer mit dem Atmungszustand des Körpers (4) des Patienten korrespondierenden Körperaktivität aufweist, und wobei der Sensor (10, 11, 12, 13) oder die Sensoranordnung (6) dazu ausgebildet ist, dass er/sie ein der Aktivität des Körpers (4) entsprechendes Sensorsignal (7) bestimmt und/oder ausgibt,
wobei das System (1) eine wiederverwendbare Zusatzvorrichtung (20) aufweist, die an dem Pflaster (2) angebracht und davon abgenommen werden kann, und
wobei die Zusatzvorrichtung (20) geeignet ist, dem Pflaster (20) drahtlos Energie zuzuführen.

2. System (1) nach Anspruch 1, **dadurch gekennzeichnet, dass** die Zusatzeinrichtung (20) eine flexible Trägerschicht umfasst, wobei ihre Komponenten auf der Trägerschicht angeordnet oder in diese eingebettet sind, und/oder dass die Zusatzeinrichtung (20) dazu eingerichtet ist, das Sensorsignal (7) oder Teile davon vom Pflaster (2) zu empfangen.

3. System (1) nach Anspruch 1 oder 2, **dadurch gekennzeichnet, dass** der Sensor (10, 11, 12, 13) ein Dehnungssensor (10) zum Erfassen einer Dehnung der Haut (3) ist oder die Sensoranordnung (6) einen Dehnungssensor (10) aufweist, wobei das Sensorsignal (7) den Grad der Dehnung repräsentiert, und/oder dass das System (1) einen Schallsensor (18), der zum Erfassen von durch Atemluftstrom (8) verursachtem Schall ausgebildet ist, und/oder einen Bewegungssensor (19) zum Erfassen einer Bewegung des Körpers (4), insbesondere der Haut (3), aufweist, wobei das Sensorsignal (7) vorzugsweise den Schall und/oder die Bewegung repräsentiert.

4. System (1) nach Anspruch 3, **dadurch gekennzeichnet, dass** das System (1) dazu ausgebildet konfiguriert ist, dass es durch Analyse der Dehnung des Körpers (4), des durch den Atemluftstrom (8) verursachten Geräusches und/oder der Bewegung des Körpers (4) einen Atemluftstromindikator (9) ermittelt oder berechnet.

5. System (1) nach einem der vorhergehenden Ansprüche, **dadurch gekennzeichnet, dass** eine mechanische, elektrische und/oder Datenverbindung zwischen dem Zusatzgerät (20) und dem Pflaster (2) hergestellt wird, wenn das Zusatzgerät (20) mit dem Pflaster (2) verbunden wird, und getrennt wird, wenn das Zusatzgerät (20) von dem Pflaster (2) getrennt wird.

6. System (1) nach einem der voranstehenden Ansprüche, **dadurch gekennzeichnet, dass** die Zusatzvorrichtung (20) so konfiguriert ist, dass sie das Sensorsignal (7) von dem Pflaster (2) drahtlos empfängt.

7. System (1) nach einem der voranstehenden Ansprüche, **dadurch gekennzeichnet, dass** das System (1) eine Verarbeitungseinrichtung (26) aufweist, die zur Berechnung eines Atemluftstromindikators (9) auf der Grundlage des Sensorsignals (7), vorzugsweise durch Korrelation des Sensorsignals (7) mit einem oder mehreren Referenzsignalen (43) oder -werten, eingerichtet und konfiguriert ist, vorzugsweise wobei die Verarbeitungseinrichtung (26) auf dem Pflaster (2) und/oder dem Zusatzgerät (20) angeordnet ist oder einen Teil davon bildet.

8. System (1) nach Anspruch 7, **dadurch gekennzeichnet, dass** die Verarbeitungsvorrichtung (26) zur Bestimmung oder Berechnung einer Atemluftstrombegrenzung, einer Änderung derselben oder einer anderen Unregelmäßigkeit auf der Grundlage des Atemluftstromindikators (9) ausgebildet ist, wobei die Verarbeitungsvorrichtung (26) vorzugsweise ferner dazu ausgebildet ist, eine Warnung (41) und/oder Anweisungen (42) zu erzeugen, wenn die Atemluftstrombegrenzung erkannt wird oder einen Schwellenwert überschreitet.

9. System (1) nach einem der voranstehenden Ansprüche, **dadurch gekennzeichnet, dass** das System (1) mindestens ein Benutzergerät (29) aufweist, wobei das Sensorsignal (7), ein Atemluftstromindikator (9), eine Warnung (41) und/oder Anweisungen (42) - vorzugsweise drahtlos - an das Benutzergerät (29) übertragbar sind; und/oder wobei das Benutzergerät (29) eine Benutzerschnittstelle (31) aufweist, die zur Ausgabe eines Atemluftstromindikators (9), einer Warnung (41) und/oder von Anweisungen (42) mittels dieser Benutzerschnittstelle (31) ausgebildet ist.

10. System (1) nach einem der vorhergehenden Ansprüche, **dadurch gekennzeichnet, dass** die Zusatzeinrichtung (20) als flexibles Pflaster ausgebildet ist und/oder zumindest im Wesentlichen an das Pflaster (2) angepasst oder ähnlich geformt ist.

11. System (1) nach einem der voranstehenden Ansprüche, **dadurch gekennzeichnet, dass** das Patch (2) eine Patch-Schnittstelle (14) zum Empfangen und/oder Senden von Energie und/oder Informationen, insbesondere eine Energiekopplungsvorrichtung (15), eine Datenkopplungsvorrichtung (16) und/oder eine Antenne (17), aufweist.

12. System (1) nach einem der voranstehenden Ansprüche, **dadurch gekennzeichnet, dass** die Zusatzeinrichtung (20) eine Zusatzeinrichtungsschnittstelle (21) aufweist, die zum Senden und/oder Empfangen des Sensorsignals (7), eines Atemluftstromindikators (9), einer Warnung (41) und/oder von Anweisungen (42), vorzugsweise drahtlos, und/oder zum elektrischen und/oder elektronischen Verbinden der Zusatzeinrichtung (20) mit dem Pflaster (2), insbesondere einer Energiekopplungseinrichtung (22), einer Datenkopplungseinrichtung (23) und/oder einer Antenne (24), ausgebildet ist.

13. System (1) nach Anspruch 11 oder 12, **dadurch gekennzeichnet, dass** die Leistungskopplungsvorrichtung(en) (15, 22) und/oder Datenkopplungsvorrichtung(en) (16, 23) zur induktiven, kapazitiven und/oder Nahfeld-Leistungsübertragung ausgebildet ist/sind.

14. Verfahren zur Erleichterung der Erfassung eines Atmungszustandes unter Verwendung eines Pflasters (2), das zur Befestigung oder Anbringung auf der Haut (3) eines Patientenkörpers (4) mittels einer Klebeschicht (2B) ausgebildet ist, wobei das Pflaster (2) mindestens einen Sensor (10, 11, 12, 13), vorzugsweise einer Sensoranordnung (6), aufweist, wobei das Pflaster (2) mindestens einen Sensor (10, 11, 12, 13), vorzugsweise eine Sensoranordnung (6), zum Erfassen einer dem Atmungszustand des Körpers (4) des Patienten entsprechenden Körperaktivität aufweist und wobei der Sensor (10, 11, 12, 13) oder die Sensoranordnung (6) zum Ermitteln und/oder Ausgeben eines der Aktivität des Körpers (4) entsprechenden Sensorsignals (7) ausgebildet ist, und
verwenden einer wiederverwendbaren Zusatzvorrichtung (20), die an dem Pflaster (2) angebracht und von diesem abgenommen werden kann,
wobei Energie dem Pflaster (2) durch die Zusatzvorrichtung (20) auf drahtlose Weise zugeführt wird.

## Revendications

1. Système (1) pour faciliter la détection d'un état respiratoire, dans lequel le système (1) comprend un patch flexible (2) configuré pour être fixé ou attaché à une peau (3) du corps d'un patient (4) au moyen d'une couche adhésive (2B), dans lequel le patch (2) comprend au moins un capteur (10, 11, 12, 13), de préférence d'un agencement de capteurs (6), pour détecter une activité corporelle correspondant à l'état respiratoire dudit corps de patient (4), et dans lequel le capteur (10, 11, 12, 13) ou l'agencement de capteurs (6) est configuré pour déterminer et/ou émettre un signal de capteur (7) correspondant à ladite activité corporelle (4),
le système (1) comprenant un dispositif d'extension réutilisable (20) pouvant être attaché au patch (2) et détaché de celui-ci, et
le dispositif complémentaire (20) étant adapté pour fournir de l'énergie au timbre (2) d'une manière sans fil.

2. Système (1) selon la revendication 1, **caractérisé en ce que** le dispositif complémentaire (20) comprend une couche de support flexible, ses composants étant situés sur ou intégrés dans la couche de support, et/ou **en ce que** le dispositif complémentaire (20) est adapté pour recevoir le signal du capteur (7) ou des parties de celui-ci à partir du patch (2).

3. Système (1) selon la revendication 1 ou 2, **caractérisé en ce que** le capteur (10, 11, 12, 13) est ou l'agencement de capteurs (6) comprend un capteur d'étirement (10) pour détecter un étirement de la peau (3), de préférence dans lequel le signal de capteur (7) représente le degré d'étirement, et/ou **en ce que** le système (1) comprend un capteur sonore (18) configuré pour détecter un son provoqué par un flux d'air respiratoire (8) et/ou un capteur de mouvement (19) pour détecter un mouvement du corps (4), en particulier de la peau du corps (3), le signal du capteur (7) représentant de préférence le son et/ou le mouvement.

4. Système (1) selon la revendication 3, **caractérisé en ce que** le système (1) est configuré pour déterminer ou calculer un indicateur de débit d'air respiratoire (9) en analysant l'étirement du corps (4), le son provoqué par le débit d'air respiratoire (8), et/ou le mouvement du corps (4).

5. Système (1) selon l'une quelconque des revendications précédentes, **caractérisé en ce qu'**une connexion mécanique, électrique et/ou de données entre le dispositif additionnel (20) et le patch (2) est créée lorsque le dispositif additionnel (20) est connecté au patch (2) et déconnectée lorsque le dispositif additionnel (20) est séparé du patch (2).

6. Système (1) selon l'une quelconque des revendications précédentes, **caractérisé en ce que** le dispositif additionnel (20) est configuré pour recevoir le signal de capteur (7) du patch (2) de manière sans fil.

7. Système (1) selon l'une quelconque des revendications précédentes, **caractérisé en ce que** le système (1) comprend un dispositif de traitement (26) étant agencé et configuré pour calculer un indicateur de débit d'air respiratoire (9) sur la base du signal de capteur (7), de préférence par corrélation du signal de capteur (7) avec un ou plusieurs signaux (43) ou valeurs de référence, de préférence dans lequel le dispositif de traitement (26) est agencé sur ou fait partie du patch (2) et/ou du dispositif additionnel (20).

8. Système (1) selon la revendication 7, **caractérisé en ce que** le dispositif de traitement (26) est configuré pour la détermination ou le calcul d'une limitation du débit d'air respiratoire, d'un changement de celle-ci, ou d'une irrégularité différente sur la base de l'indicateur de débit d'air respiratoire (9), de préférence dans lequel le dispositif de traitement (26) est en outre configuré pour générer un avertissement (41) et/ou des instructions (42) si la limitation du débit d'air respiratoire est détectée ou dépasse un seuil.

9. Système (1) selon l'une quelconque des revendications précédentes, **caractérisé en ce que** le système (1) comprend au moins un dispositif utilisateur (29), dans lequel le signal de capteur (7), un indicateur de débit d'air respiratoire (9), un avertissement (41) et/ou des instructions (42) peuvent être transmis - de préférence de manière sans fil - au dispositif utilisateur (29) ; et/ou dans lequel le dispositif utilisateur (29) comprend une interface utilisateur (31) qui est configurée pour émettre un indicateur de débit d'air respiratoire (9), un avertissement (41) et/ou des instructions (42) au moyen de cette interface utilisateur (31).

10. Système (1) selon l'une quelconque des revendications précédentes, **caractérisé en ce que** le dispositif complémentaire (20) est formé comme un patch flexible et/ou est au moins essentiellement adapté ou formé de manière similaire au patch (2).

11. Système (1) selon l'une quelconque des revendications précédentes, **caractérisé en ce que** le patch (2) comprend une interface de patch (14) pour recevoir et/ou émettre de l'énergie et/ou des informations, notamment un dé-verseur de couplage de puissance (15), un dispositif de couplage de données (16) et/ou une antenne (17).

12. Système (1) selon l'une quelconque des revendications précédentes, **caractérisé en ce que** le dispositif additionnel (20) comprend une interface de dispositif additionnel (21) configurée pour transmettre et/ou recevoir le signal de capteur (7), un indicateur de débit d'air respiratoire (9), un avertissement (41), et/ou des instructions (42), de préférence sans fil, et/ou pour connecter le dispositif additionnel (20) au patch (2) électriquement et/ou électroniquement, notamment un dispositif de couplage de puissance (22), un dispositif de couplage de données (23) et/ou une antenne (24).

13. Système (1) selon la revendication 11 ou 12, **caractérisé en ce que** le ou les dispositifs de couplage de puissance (15, 22) et/ou le ou les dispositifs de couplage de données (16, 23) sont configurés pour un transfert de puissance inductif, capacitif et/ou en champ proche.

14. Procédé pour faciliter la détection d'un état respiratoire en utilisant un patch (2) configuré pour être apposé ou attaché à la peau (3) du corps d'un patient (4) au moyen d'une couche adhésive (2B), dans lequel le patch (2) comprend au moins un capteur (10, 11, 12, 13), de préférence d'un arrangement de capteurs (6), pour détecter une activité corporelle correspondant à l'état respiratoire du corps (4) dudit patient et dans lequel le capteur (10, 11, 12, 13) ou l'agencement de capteurs (6) est configuré pour déterminer et/ou émettre un signal de capteur (7) correspondant à ladite activité corporelle (4), et
en utilisant un dispositif complémentaire réutilisable (20) qui peut être attaché au patch (2) et détaché de celui-ci,
dans lequel l'énergie est fournie au patch (2) par le dispositif complémentaire (20) d'une manière sans fil.
